(19) **Europäisches Patentamt European Patent Office Office européen des brevets**

(11) **EP 4 463 226 B1**

(12) **FASCICULE DE BREVET EUROPEEN**

(45) Date de publication et mention de la délivrance du brevet:
**05.11.2025 Bulletin 2025/45**

(21) Numéro de dépôt: **22850653.1**

(22) Date de dépôt: **16.12.2022**

(51) Classification Internationale des Brevets (IPC):
**A61N 7/02** *(2006.01)* **A61B 34/10** *(2016.01)*

(52) Classification Coopérative des Brevets (CPC):
**A61B 34/10; A61N 7/02;** A61B 2034/104;
A61B 2034/105; A61N 2007/0095

(86) Numéro de dépôt international:
**PCT/FR2022/052404**

(87) Numéro de publication internationale:
**WO 2023/111487 (22.06.2023 Gazette 2023/25)**

(54) **MÉTHODE ET SYSTÈME DE PARAMÉTRAGE D'UN DISPOSITIF DE TRAITEMENT PAR ULTRASONS FOCALISÉS DE HAUTE INTENSITÉ**

VERFAHREN UND SYSTEM ZUR PARAMETRISIERUNG EINER HOCHINTENSIVEN FOKUSSIERTEN ULTRASCHALLBEHANDLUNGSVORRICHTUNG

METHOD AND SYSTEM FOR PARAMETERISING A HIGH-INTENSITY FOCUSED ULTRASOUND TREATMENT DEVICE

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **17.12.2021 FR 2113901**

(43) Date de publication de la demande:
**20.11.2024 Bulletin 2024/47**

(73) Titulaires:
• **Commissariat à l'Energie Atomique et aux Energies Alternatives**
**75015 Paris (FR)**
• **Edap-TMS**
**69120 Vaulx en Velin (FR)**

(72) Inventeurs:
• **CHATILLON, Sylvain**
**91120 PALAISEAU (FR)**
• **CARDOSO, Michel**
**92100 BOULOGNE-BILLANCOURT (FR)**
• **GUILLEN, Nicolas**
**69270 FONTAINES SUR SAONE (FR)**

(74) Mandataire: **Brevalex**
**Tour Trinity**
**1 B Place de la Défense**
**92400 Courbevoie (FR)**

(56) Documents cités:
**WO-A1-2019/053614 US-A1- 2014 296 842**

• **CHATILLON S ET AL: "Applications of intensive HIFU simulation based on surrogate models using the CIVA HealthCare platform", JOURNAL OF PHYSICS: CONFERENCE SERIES, INSTITUTE OF PHYSICS PUBLISHING, BRISTOL, GB, vol. 1761, no. 1, 26 January 2021 (2021-01-26), pages 12007, XP020363469, ISSN: 1742-6596, [retrieved on 20210126], DOI: 10.1088/1742-6596/1761/1/012007**
• **SAPARETO S A ET AL: "Thermal dose determination in cancer therapy", INTERNATIONAL JOURNAL OF RADIATION: ONCOLOGY BIOLOGY PHYSICS, PERGAMON PRESS, USA, vol. 10, no. 6, 1 April 1984 (1984-04-01), pages 787 - 800, XP026841823, ISSN: 0360-3016, [retrieved on 19840401]**

**(Cont. page suivante)**

• LIU XILUN ET AL: "An Optimized Control Approach for HIFU Tissue Ablation Using PDE Constrained Optimization Method", IEEE TRANSACTIONS ON ULTRASONICS, FERROELECTRICS, AND FREQUENCY CONTROL, IEEE, USA, vol. 68, no. 5, 25 November 2020 (2020-11-25), pages 1555 - 1568, XP011852061, ISSN: 0885-3010, [retrieved on 20210423], DOI: 10.1109/TUFFC.2020.3040362

## Description

## DOMAINE TECHNIQUE

La présente invention concerne de manière générale les dispositifs de traitement par ultrasons focalisés de haute intensité ou HIFU *(High Intensity Focused Ultrasound)*. Elle trouve notamment application à la destruction tumorale par ablation thermique à distance avec préservation de tissus intermédiaires.

## ÉTAT DE LA TECHNIQUE ANTÉRIEURE

La thérapie par ultrasons focalisés de haute intensité (HIFU) consiste à utiliser l'énergie d'un faisceau ultrasonore focalisé, pour modifier ou détruire des tissus biologiques. La destruction tissulaire intervient par coagulation protéique induisant des lésions cellulaires irréversibles et l'apoptose.

Cette technique est largement utilisée pour l'ablation thermique de tumeurs bénignes ou malignes (cancer de la prostate, métastases hépatiques, tumeurs cérébrales). Elle présente l'avantage d'être percutanée (donc non invasive) et de permettre la préservation des tissus sains environnants.

Le traitement par HIFU fait généralement appel à une sonde multi-transducteur à réseau de phase *(phased array)*. A chaque tir ultrasonore, une loi de retards et une loi d'amplitudes sont appliquées aux différents éléments du transducteur, de sorte à former un faisceau focalisé en un point prédéterminé. L'opération est répétée en différents points de la zone cible à détruire, en choisissant la cadence de répétition (fréquence de récurrence) et l'énergie des impulsions.

Avant d'appliquer le protocole de traitement, le praticien doit s'assurer que la dose thermique déposée est suffisante pour détruire la zone cible tout en préservant les zones saines. Pour ce faire, la zone cible est maillée par une grille de points et une simulation de tirs ultrasonores focalisés en chaque point ou un groupe de points voisins de la grille est réalisée. L'élévation de température en chaque point est ensuite déduite à partir de la résolution de l'équation de transfert thermique dans les tissus. Une méthode de validation d'un protocole de traitement par HIFU a été décrite dans la demande US-A-2021/0000541.

Cette méthode de simulation fonctionne relativement bien lorsque le milieu dans lequel se propagent les ondes ultrasonores est homogène mais devient nettement plus complexe dès lors que plusieurs zones tissulaires sont à modéliser, *a fortiori* lorsqu'une résolution spatiale élevée est nécessaire. La simulation du champ ultrasonore à partir des caractéristiques des différents tissus du patient peut alors nécessiter plusieurs heures sur un ordinateur personnel conventionnel, ce qui est incompatible avec un traitement ambulatoire.

Il a par ailleurs été proposé d'accélérer notablement la simulation d'un traitement HIFU grâce à l'utilisation d'un métamodèle généré à partir d'une base de données simulée. Celle-ci est obtenue en définissant un ensemble de N paramètres incertains, ici les caractéristiques physiologiques et anatomiques du patient influents pour la simulation, ainsi que leur domaine de variation supposé. La base de données est obtenue en effectuant une fois pour toutes *(off-line)* la simulation du champ ultrasonore pour l'ensemble des configurations possibles dans cet espace de dimension N (configurations de base). Cette première simulation donne la distribution du champ de pression ultrasonore pour chaque configuration de base.

Une seconde simulation est ensuite effectuée en ligne *(on line)* en interpolant les résultats de simulation obtenus pour les différentes configurations précédentes, c'est-à-dire en interpolant les champs de pression ultrasonores contenus dans la base de données. On parle alors de méta-modèle puisqu'il s'agit de remplacer le modèle de simulation direct, supposé exact par un interpolateur beaucoup plus rapide, voire temps réel, dont on maîtrise l'erreur.

Une telle méthode de simulation d'un champ ultrasonore HIFU a été décrite dans l'article de S. Chatillon et al., intitulé « Applications of intensive HIFU simulation based on surrogate models using the CIVA HealthCare platform » publié dans J. of Phys., 1761, 2021.

Si les paramètres physiologiques et anatomiques du patient sont parfaitement connus, cette méthode de simulation permet d'estimer la lésion réalisée pour un protocole établi, et ainsi de vérifier qu'il respecte les contraintes imposées en termes de zone cible à détruire et de tissus sains à préserver. Lorsque le protocole de traitement envisagé par le praticien ne respecte pas les contraintes précitées, celui-ci doit le modifier de manière empirique et effectuer une nouvelle simulation en ligne. La convergence du processus peut être relativement lente et conduire à une solution sous-optimale.

Le but de la présente invention est par conséquent de proposer une méthode et un système de paramétrage embarqués au sein d'un dispositif de traitement HIFU qui ne présente pas les inconvénients précités, en particulier qui permette d'obtenir très rapidement un paramétrage quasi-optimal à partir des paramètres physiologiques du patient, de la zone cible à nécroser et des zones saines à épargner.

Par ailleurs le document US2014296842 A1 divulgue une méthode où la distribution de la température spécifique au patient dans les organes, due à un dispositif d'ablation, est simulée. Les effets de l'ablation sont modélisés. La modélisation est spécifique au patient. La structure des vaisseaux d'un patient donné, segmentée à partir d'images médicales, est prise en compte en tant que puits de chaleur dans le modèle de transfert de chaleur biologique. Une carte de température est générée pour montrer les effets de l'ablation dans une analyse préopératoire. Les cartes de température résultant de différents courants d'ablation et positions du dispositif d'ablation peuvent être utilisées pour déterminer un emplacement optimal du dispositif d'ablation pour un patient donné. D'autres modèles peuvent être inclus, tels que la prise en compte des lésions tissulaires au cours de l'ablation.

## EXPOSÉ DE L'INVENTION

La présente invention est définie par une méthode de paramétrage d'un dispositif de traitement HIFU équipé d'une sonde ultrasonore pour nécroser au moins une région tissulaire d'une zone à traiter d'un patient, ledit traitement étant original en ce qu'il comprend une pluralité de tirs ultrasonores et étant original en ce que les paramètres de traitement définissant notamment la position et l'orientation de la sonde à chaque tir relativement à la zone à traiter, la zone à traiter étant modélisée par différentes régions tissulaires, incluant au moins une région tissulaire à nécroser et une région tissulaire à préserver, chaque région tissulaire étant caractérisée par des paramètres géométriques et des paramètres physiologiques, ladite méthode de paramétrage comprenant :

- une étape d'estimation du champ ultrasonore dans la zone à traiter au moyen d'un méta-modèle utilisant une base de données dans laquelle sont stockées des cartographies précalculées de champ ultrasonore pour différentes valeurs des paramètres géométriques et physiologiques de chaque région tissulaire de ladite zone, l'estimation du champ ultrasonore étant obtenue par interpolation de cartographies précalculées ;
- une étape d'estimation de la dose thermique déposée en chaque point d'une grille au sein de la zone à traiter à partir du champ ultrasonore dans cette zone ;
- une étape d'estimation de la réponse tissulaire en chaque point de ladite grille pour déterminer si la dose thermique déposée conduit ou non à une nécrose en ce point, définissant ainsi une région nécrosée estimée et une région préservée estimée ;
- une étape d'adaptation des paramètres de traitement visant à minimiser une fonction de coût dépendant de l'écart entre la région nécrosée estimée et de la région tissulaire à nécroser, sous contrainte que la région à préserver soit comprise dans la région préservée estimée.

Lesdits paramètres de traitement peuvent également comprendre la durée et la cadence de répétition des tirs ultrasonores.

Lorsque la sonde comprend une pluralité de transducteurs, lesdits paramètres de traitement peuvent comprendre une loi de phase et/ou de fréquence et/ou de puissance à appliquer sur l'ensemble des éléments transducteurs.

Les paramètres physiologiques d'une région tissulaire comprennent par exemple au moins un paramètre parmi la densité, la chaleur spécifique, la conductivité thermique du tissu, la chaleur spécifique du sang et le taux de perfusion sanguine du tissu.

L'estimation du champ ultrasonore peut être obtenue dans le repère local de la sonde par interpolation desdites cartographies précalculées, la distribution du champ ultrasonore dans la zone à calculer en étant déduite par changement de repère.

L'estimation de la dose thermique est avantageusement obtenue par une résolution d'une équation de transfert de chaleur dans les différentes régions tissulaires, prenant en compte l'énergie ultrasonore absorbée en chaque point de la grille à chaque tir ultrasonore.

Le résultat du traitement peut être simulé en calculant, en chaque point de la grille, la dose thermique absorbée en ce point sous la forme d'une durée d'exposition équivalente à une température de référence, le tissu étant considéré comme nécrosé en ce point si cette durée est supérieure à une valeur de seuil prédéterminée et préservé sinon.

Selon une variante, la fonction de coût dépend de l'écart entre l'étendue de la région tissulaire à nécroser avec celle de la région tissulaire nécrosée estimée.

La fonction de coût peut être définie de différentes manières.

Selon une variante, la fonction de coût est définie comme une somme de fonctions de coût élémentaires associées à chacun des tirs.

Selon certaines variantes, la fonction de coût inclut une somme d'une pluralité de fonctions de coût élémentaires, chacune desdites fonctions de coût élémentaire étant associée à un tir, à une région tissulaire à nécroser, ou à une sous-région d'une région tissulaire à nécroser, et/ou étant une fonction visant à minimiser ou maximiser au moins un objectif choisi parmi

- un écart entre une région ou une sous-région de celle-ci estimée nécrosée par un ou plusieurs tirs et la région ou la sous-région de celle-ci à nécroser correspondante,
- un volume de tissus sains nécrosés à l'issue d'un ou plusieurs tirs,
- une durée globale d'un ou plusieurs tirs,
- un nombre de positions de la sonde pour la réalisation d'un ensemble de tirs, notamment de la totalité des tirs,
- une période de repos entre deux tirs consécutifs ou une somme de telles périodes de repos.

Dans cette somme, une ou certaines fonctions de coût élémentaire peuvent également être une ou des fonctions résultant de l'ensemble des tirs prévus par le traitement, visant donc notamment à couvrir l'ensemble de la ou des régions tissulaires à nécroser.

Une sous-région d'une région tissulaire à nécroser est une partie de cette région.

Lorsque des fonctions de coût élémentaires sont associées respectivement à différents tirs, de préférence ces différents tirs sont dirigés vers des sous-régions de la région tissulaire à nécroser distinctes les unes des autres.

Lorsqu'une fonction de coût élémentaire est associée à une sous-région de la région à nécroser, il peut être prévu un tir (unique) ou une pluralité de tirs pour la sous-région considérée.

Dans certaines variantes, chacune des fonctions de coût élémentaire est associée respectivement à l'un des différents tirs, les différents tirs étant dirigés de préférence vers des sous-régions distinctes les unes des autres de la région à nécroser.

Dans certaines variantes, chacune des fonctions de coût élémentaire est associée respectivement à une sous-région de la région à nécroser, un tir ou une pluralité de tirs étant prévu(e) pour chaque sous-région de la région à nécroser.

En outre, dans certaines variantes, la fonction de coût est définie par une somme de fonctions de coût élémentaires, chacune des fonctions de coût élémentaires visant à minimiser au moins un objectif à minimiser choisi parmi les objectifs indiqués ci-dessus.

Selon certaines variantes, les paramètres de traitement sont adaptés de manière itérative au moyen d'une descente de gradient, d'une descente de gradient stochastique ou d'un algorithme génétique.

Pour au moins une itération d'adaptation des paramètres de traitement, la région tissulaire nécrosée estimée et/ou la région tissulaire préservée estimée sont/est avantageusement visualisée(s) sur un afficheur avec la région tissulaire à nécroser et/ou la région tissulaire à préserver en superposition avec l'image de la zone à traiter.

La présente invention concerne également un système de paramétrage d'un dispositif de traitement HIFU équipé d'une sonde ultrasonore pour nécroser au moins une région tissulaire d'une zone à traiter d'un patient, ledit traitement comprenant une pluralité de tirs ultrasonores et étant caractérisé par des paramètres de traitement définissant notamment la position et l'orientation de la sonde à chaque tir relativement à la zone à traiter, la zone à traiter étant modélisée par différentes régions tissulaires, incluant au moins un région tissulaire à nécroser et une région tissulaire à préserver, chaque région tissulaire étant caractérisée par des paramètres géométriques et physiologiques, ledit système de paramétrage comprenant :

- un module d'estimation du champ ultrasonore dans la zone à traiter au moyen d'un méta-modèle, une base de données dans laquelle sont stockées des cartographies précalculées de champ ultrasonore pour différentes valeurs des paramètres géométriques et physiologiques de chaque région tissulaire de ladite zone, ledit module d'estimation estimant le champ ultrasonore par interpolation de cartographies précalculées extraites de la base de données ;
- un module d'estimation de la dose thermique déposée en chaque point d'une grille au sein de la zone à traiter à partir du champ ultrasonore dans cette zone ;
- un module d'estimation de la réponse tissulaire en chaque point de ladite grille pour déterminer si la dose thermique déposée conduit ou non à une nécrose en ce point, définissant ainsi une région nécrosée estimée et une région préservée estimée ;
- un module d'adaptation des paramètres de traitement pour minimiser une fonction de coût dépendant de l'écart entre la région nécrosée estimée et de la région tissulaire à nécroser, sous contrainte que la région à préserver soit comprise dans la région préservée estimée.

Lesdits paramètres de traitement comprennent également la durée et la cadence de répétition des tirs ultrasonores.

Lorsque la sonde comprend une pluralité de transducteurs, lesdits paramètres de traitement peuvent comprendre une loi de phase et/ou de fréquence et/ou de puissance à appliquer sur l'ensemble des éléments transducteurs.

BRÈVE DESCRIPTION DES DESSINS

D'autres caractéristiques et avantages de l'invention apparaîtront à la lecture d'un mode de réalisation préférentiel de l'invention, fait en référence aux figures jointes parmi lesquelles :

La Fig. 1 représente de manière schématique un système de paramétrage d'un dispositif de traitement HIFU selon un mode de réalisation de l'invention ;
La Fig. 2 représente de manière schématique le module de simulation utilisé dans le système de paramétrage de la Fig. 1 ;
La Fig. 3 représente de manière schématique une méthode de prédiction de la distribution de l'intensité du champ ultrasonore, utilisée dans la Fig. 2 ;
La Fig. 4 représente de manière schématique une méthode d'estimation de la dose thermique déposée dans la zone à traiter, utilisée dans la Fig. 2.

**EXPOSÉ DÉTAILLÉ DE MODES DE RÉALISATION PARTICULIERS**

Nous considérerons dans la suite un dispositif de traitement par ultrasons focalisés de haute intensité tel que décrit dans la partie introductive. Un tel dispositif peut être équipé d'une sonde à transducteur piézoélectrique unique dont la forme donne la loi de focalisation, ou plus généralement une sonde multi-transducteur, la loi de focalisation étant donnée par la distribution des retards et des puissances acoustiques relatifs aux différents éléments piézo-électriques. Dans la suite, nous supposerons dans un but de simple illustration et sans préjudice de généralisation que la sonde est multi-transducteur. Le traitement est réalisé par des tirs successifs, chaque tir correspondant à l'application d'une distribution donnée de phases, d'amplitudes, voire de fréquences. La présence de milieux hétérogènes entre le transducteur et la zone cible pourront donner lieu à l'application, sur chaque élément transducteur, d'une valeur spécifique de phase, d'amplitude, ou de fréquence. Par exemple, l'absorption par un milieu de forte atténuation acoustique (organe ou tumeur par exemple) pourra être compensée par une augmentation de la puissance sur certains éléments transducteurs ou la diminution de la fréquence d'émission, de manière à équilibrer les contributions énergétiques au point focal.

L'idée à la base de la présente invention est de prévoir un module (plug-in) qui puisse être adjoint à ou intégré dans un dispositif de traitement HIFU de manière à fournir un paramétrage de la sonde à ultrasons pour une zone à traiter donnée.

Plus précisément, le praticien définit préalablement une zone à traiter dans le corps du patient, cette zone comprenant des parties à nécroser (tissus cancéreux, métastases etc.) par effet thermique (élévation de la température locale à une valeur de seuil prédéterminée pendant une durée donnée) et des parties à préserver (tissus sains).

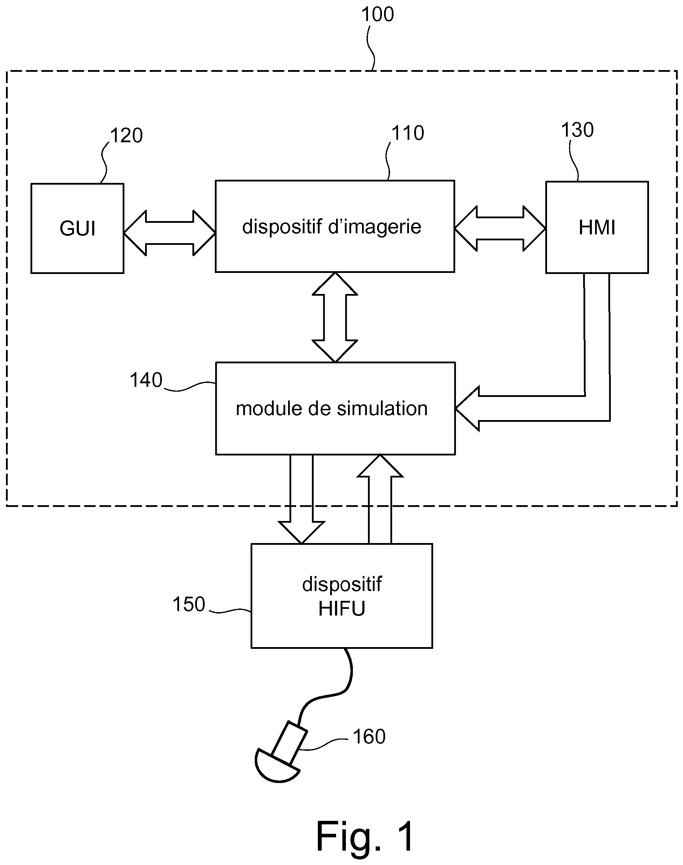

Un dispositif de traitement HIFU associé à un système de paramétrage selon la présente invention a été représenté schématiquement en Fig. 1.

On a représenté en 150 le dispositif HIFU équipé de sa sonde à ultrasons 160.

Le système de paramétrage 100 peut recevoir du dispositif HIFU des données représentant la zone à traiter ainsi que les caractéristiques de la sonde et lui fournir en retour les paramètres pour insonifier la zone en question.

Le système de paramétrage comprend essentiellement un module de simulation 140 permettant d'estimer pour chaque tir l'intensité du champ ultrasonore dans la zone à traiter et d'en déduire la dose thermique déposée en chaque point cette zone.

Le système de paramétrage pourra avantageusement être équipé d'un dispositif d'imagerie, 110, d'une interface utilisateur, 130, et d'une interface graphique, 120.

Le praticien pourra visualiser la zone à traiter sur l'afficheur du dispositif d'imagerie et planifier le traitement sous la forme d'une séquence de tirs, chaque tir étant défini par une disposition de la sonde par rapport à la zone à traiter (position et orientation de la sonde relativement à cette dernière), un point focal, autrement dit une loi de retard des impulsions appliquées aux différents éléments de la sonde, une énergie d'impulsion (puissance et durée, le cas échéant), voire une fréquence ultrasonore.

Alternativement, la séquence de tirs pourra avoir été définie préalablement par le praticien à l'aide du dispositif HIFU et les données correspondantes auront été transmises au système de paramétrage. Ce pourra notamment être le cas si un traitement précédent du patient a été enregistré dans le dispositif HIFU.

En tout état de cause, la zone à traiter ainsi que les tirs successifs pourront être visualisés sur l'afficheur du dispositif d'imagerie. Ce dispositif pourra être par exemple celui d'un échographe classique.

Le praticien pourra à l'aide de l'interface utilisateur définir des tirs additionnels ou supprimer des tirs programmés, modifier la position et/ou l'orientation de la sonde, régler la puissance et/ou la durée voire la fréquence de répétition des tirs etc. La séquence de tirs pourra être représentée en superposition avec l'image de la zone à traiter.

Une fois les paramètres nominaux de traitement définis (positions et orientations de la sonde, puissance et durée des tirs, fréquence ultrasonore, fréquence de répétition des tirs etc.), le module de simulation permet, comme décrit plus loin, de calculer en quasi temps réel la distribution spatiale du champ ultrasonore à chaque tir, et d'en déduire la distribution de la dose thermique dans les tissus. Cette possibilité de simulation en quasi-temps réel des effets d'une séquence de tirs est essentielle dans la mesure où elle permet un traitement en ambulatoire.

Il est alors possible de visualiser sur l'écran de l'afficheur une cartographie de la dose thermique et d'en déduire, par comparaison avec un seuil les parties nécrosées et les parties épargnées. Le praticien peut ainsi vérifier si le plan de traitement HIFU est bien conforme à l'objectif souhaité et, dans l'affirmative, valider le transfert des paramètres de traitement au dispositif HIFU.

Le module 140 peut lui-même comparer le résultat de la simulation avec l'objectif souhaité et modifier de manière itérative les paramètres du dispositif de traitement HIFU pour se conformer au plan de traitement. Par exemple, le module de simulation peut ajuster la position et/ou l'orientation de la sonde par rapport à la zone à traiter, la puissance et/ou la durée des tirs, la cadence des tirs, etc.

Dans tous les cas, la conformité du paramétrage du dispositif de traitement est validée par le praticien au moyen de l'interface utilisateur avant que les paramètres ne soient transférés au dispositif HIFU.

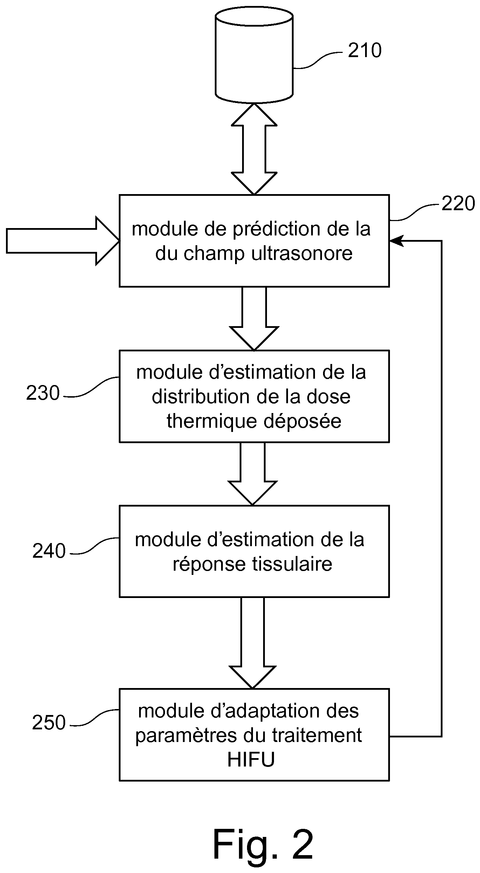

La Fig. 2 représente de manière schématique le module de simulation utilisé dans le système de paramétrage de la Fig. 1.

Ce module de simulation fait appel à un métamodèle pour prédire, à l'aide d'une base de données 210, l'intensité du champ ultrasonore dans la zone à traiter. Plus précisément, la configuration de la zone à traiter est modélisée en la décomposant en différents régions tissulaires, chaque région tissulaire étant caractérisée par des paramètres géométriques et physiologiques.

La configuration de la zone à traiter peut différer d'un patient à l'autre en fonction des caractéristiques géométriques et des paramètres physiques des régions tissulaires qui la composent. Par exemple, les régions tissulaires peuvent être de la graisse, du sang, de la peau, le parenchyme d'un organe.

Les paramètres géométriques des différentes régions tissulaires pourront être par exemple l'épaisseur, le rayon de courbure et tout autre paramètre permettant de décrire la forme ou le volume d'une telle région.

Parmi les paramètres physiologiques d'une région tissulaire, on pourra notamment prendre en compte le coefficient d'atténuation acoustique du tissu, son impédance acoustique, sa densité, sa chaleur spécifique, sa conductivité thermique, sa température, etc.

La base de données contient une cartographie de l'intensité du champ acoustique dans la zone à traiter pour une pluralité de configurations possibles de cette zone et une pluralité de paramètres de traitement. Plus précisément, pour chaque configuration de ladite pluralité, la base de données contient une cartographie du champ de pression dans la zone à traiter.

Par exemple, la base de données contiendra une cartographie du champ de pression pour différentes épaisseurs de peau, de couche de graisse, de taille ou de valeurs de paramètres géométriques et/ou physiologiques d'un organe. Cette cartographie pourra être stockée dans la base pour différents paramètres de traitement et notamment différentes positions et orientations relatives de la sonde et de la zone à traiter.

Chaque cartographie est associée à un N-uplet d'échantillons de ces N paramètres géométriques et physiques. Ainsi par exemple, pour une inclinaison donnée de la sonde, une épaisseur de peau donnée, une épaisseur de graisse donnée, une valeur d'atténuation acoustique dans un organe, la base de données contient la distribution précalculée de l'intensité du champ acoustique dans ladite zone à traiter. Pour chacune de ces configurations, cette distribution aura été elle-même été obtenue par une simulation *offline* (plateforme de simulation CIVA par exemple). Il est important de noter qu'une telle simulation requiert d'importantes ressources de calcul et est donc incompatible avec des contraintes de temps réel.

Le module de prédiction 220 extrait de la base de données, la ou les cartographies de champ ultrasonore correspondant aux paramètres géométriques et physiques les plus proches de ceux définissant le protocole de traitement envisagé. Le protocole de traitement comprend notamment les positions et orientations successives de la sonde par rapport à la zone à traiter.

Ces paramètres de traitement peuvent être des paramètres nominaux initialement fournis par le dispositif HIFU et/ou ceux corrigés par le praticien à l'aide du dispositif d'imagerie 110 et de l'interface utilisateur 130.

Le module de prédiction effectue une interpolation des cartographies de champ ultrasonore ainsi obtenues pour obtenir une cartographie correspondant aux paramètres géométriques et physiques du traitement.

Cette cartographie de champ ultrasonore est obtenue pour chaque tir dans le repère local de la sonde puis transformée par changement de repère en une cartographie dans le repère de la zone à traiter.

La cartographie de la zone à traiter est fournie au module d'estimation de dose thermique déposée, 230. Celui-ci calcule l'élévation de température induite par l'apport d'énergie ultrasonore en chaque point d'une grille de points dans la zone à traiter.

Le module d'estimation de la réponse tissulaire, 240, détermine ensuite en chaque point de la grille si la dose thermique déposée, correspondant à l'accumulation de l'échauffement thermique au cours du temps, conduit ou non à une nécrose tissulaire. Pour ce faire, ce module pourra accéder aux caractéristiques physiologiques des différents tissus stockées dans la base de données 210. Par exemple, pour un tissu donné, le franchissement d'un seuil de dose thermique déposée sera un critère permettant de conclure à sa destruction. En revanche, si ce seuil n'est pas franchi sur la durée en question, le tissu pourra être considéré comme préservé. Le cas échéant, le critère sera exprimé sous forme de probabilité de destruction en fonction de statistiques déjà obtenues sur les tissus en question.

Une cartographie des régions nécrosées ou de probabilité de nécrose tissulaire peut ensuite être superposée à l'image de la zone à traiter sur l'afficheur du dispositif d'imagerie 110.

Avantageusement, un module d'adaptation des paramètres de traitement utilisera une fonction de coût dépendant de l'écart observé en chaque point des régions à détruire. Cette fonction de coût est minimisée sous contrainte de ne pas remplir le critère de destruction dans les régions à préserver. Le cas échéant, les différentes régions à détruire seront affectées de coefficients de pondération dans la fonction de coût en fonction de l'importance de leur prise en compte dans le traitement.

La fonction de coût peut s'exprimer sous la forme d'une somme de fonctions élémentaires.

Ces fonctions élémentaires peuvent être notamment associées à chacun des tirs. Dans ce cas, chaque fonction élémentaire peut notamment dépendre de la position et de l'orientation de la sonde lors du tir, de la puissance et la durée des impulsions ainsi que la loi de focalisation appliquées (donnant la position du point focal) lors de ce tir.

Les fonctions élémentaires peuvent également être associées à des régions tissulaires à nécroser.

En particulier, dans certains cas, une région tissulaire à nécroser peut être considérée comme comprenant plusieurs sous-régions. Dans ce cas, la fonction de coût peut comprendre une somme de fonction de coût élémentaires, chaque fonction de coût élémentaire étant associée à l'une des sous-régions. Il peut naturellement être prévu un ou plusieurs tirs pour chaque région ou sous-région tissulaire à nécroser.

Indépendamment du fait qu'une fonction de coût élémentaire soit associée à un tir, à une région ou sous-région tissulaire à nécroser, ou même à l'ensemble des régions à nécroser, une fonction de coût élémentaire peut prendre en compte un ou plusieurs objectifs à optimiser. Ces objectifs peuvent être notamment :

- un écart entre une région ou une sous-région de celle-ci estimée nécrosée par un ou plusieurs tirs et la région ou la sous-région de celle-ci à nécroser correspondante,
- un volume de tissus sains nécrosés à l'issue d'un ou plusieurs tirs,
- une durée globale (cumulée) d'un ou plusieurs tirs,
- un nombre de positions de la sonde pour la réalisation d'un ensemble de tirs, notamment de la totalité des tirs, et
- une période de repos entre deux tirs consécutifs ou une somme de telles périodes de repos.

Ces objectifs sont pris en compte dans la fonction de coût afin notamment d'améliorer le confort du patient et/ou d'optimiser la disponibilité de l'appareil de traitement.

La minimisation de la fonction de coût sous contrainte peut être réalisée de manière classique avec des multiplicateurs de Lagrange, par exemple en associant un multiplicateur de Lagrange à chaque région à préserver.

La minimisation de la fonction de coût peut être réalisée au moyen d'une descente de gradient, voire une descente de gradient stochastique lorsque le nombre de tirs est important, voire des algorithmes génétiques, du type évolution différentielle par exemple

Les nouveaux paramètres de traitement obtenus sont ensuite injectés dans le module de prédiction 220 pour une nouvelle prédiction du champ ultrasonore.

Le praticien peut visualiser à chaque itération la cartographie des régions nécrosées et des zones préservées et valider le transfert des paramètres de traitement au dispositif HIFU.

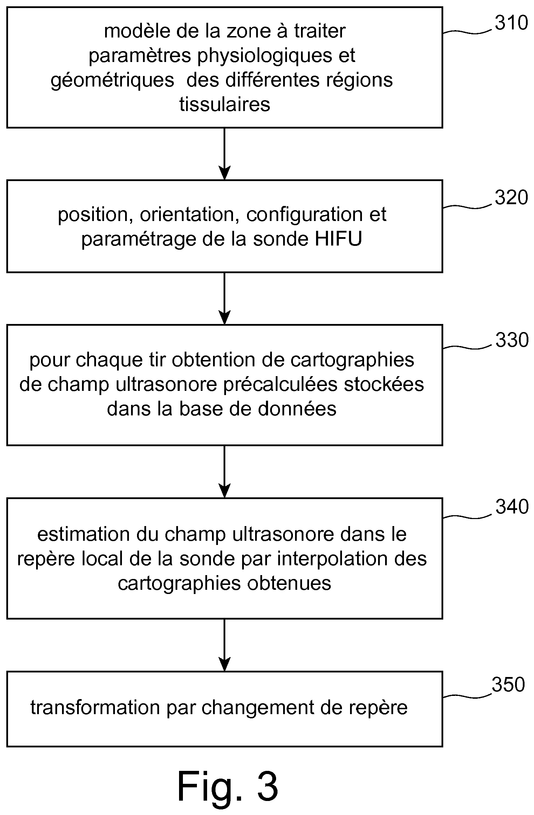

La Fig. 3 représente de manière schématique une méthode de prédiction de la distribution de l'intensité du champ ultrasonore, utilisée dans la Fig. 2.

La méthode de prédiction suppose d'avoir préalablement modélisé en 310 la zone à traiter en différentes régions tissulaires, chaque région tissulaire étant caractérisée par un paramètre géométrique et/ou physiologique comme indiqué plus haut.

Elle suppose également d'avoir modélisé en 320 le protocole de traitement au moyen de paramètres de traitement, tels que la position et l'orientation de la sonde relativement à la zone à traiter, ainsi que la puissance, la durée, la loi de retards/phases, la fréquence des impulsions appliquées aux différents éléments transducteurs de la sonde, ce à chaque tir. La séquence de tirs peut être décrite par une trajectoire de sonde relativement à la zone à traiter, cette dernière pouvant le cas échéant être divisée en tranches.

A partir des paramètres géométriques et physiologiques des régions tissulaires, ainsi que des paramètres de traitement, les configurations pertinentes de ces paramètres, par exemple les configurations les plus proches ou relatives à des valeurs appartenant à des plages autour de ces paramètres sont recherchées dans la base de données. Les cartographies précalculées de champ ultrasonore associées à ces différentes configurations sont alors extraites de la base.

En 340, la distribution de l'intensité du champ ultrasonore dans la zone à traiter est estimée dans le repère local de la sonde en interpolant les cartographies de champ extraites de la base de données. Cette estimation est répétée pour chaque tir du protocole de traitement.

Enfin, on effectue en 350 un changement de repère à chaque tir de manière à obtenir la distribution du champ ultrasonore dans le repère de la zone à traiter.

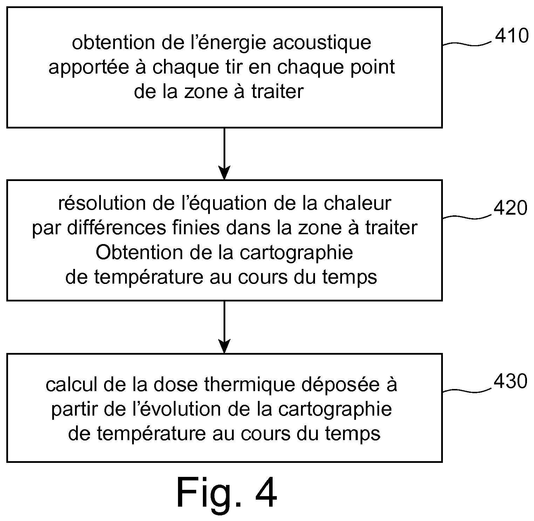

La Fig. 4 représente de manière schématique une méthode d'estimation de la dose thermique déposée dans la zone à traiter, utilisée dans la Fig. 2.

La zone à traiter est échantillonnée spatialement au moyen d'une grille de points.

A chaque tir, on estime en 410, pour chaque point de la grille l'énergie acoustique apportée en chaque point, compte tenu de la puissance et de la durée du tir et de l'atténuation le long des trajets de propagation dans les différentes régions tissulaires.

A l'étape 420, l'équation de transfert de chaleur dans les tissus biologiques ou BHTE (*Bio Heat Transfer Equation*) est résolue au moyen d'une méthode de résolution explicite par différences finies. On pourra trouver une présentation de cette méthode de résolution dans l'ouvrage de J. Chato intitulé « Fundamentals of Bioheat Transfer ; Springer Berlin, Heidelberg, 1990.

L'équation BHTE peut s'exprimer sous la forme suivante :

$$\rho.C\frac{\delta T_P}{\delta t} = \nabla.k\nabla T_P + \omega_b C_b \left(T_A - T_P\right) + Q_P$$

où $T_P$ est la température au point P et à l'instant $t$, $T_A$ est la température du sang artériel, $\rho$, $C$ et $k$ sont respectivement la densité , la chaleur spécifique du tissu et la conductivité thermique du tissu, $C_b$ et $\omega_b$ sont respectivement la chaleur spécifique du sang et le taux de perfusion sanguine, et enfin $Q_P$ est la densité d'énergie ultrasonore absorbée au point $P$.

Les conditions initiales sont données par la température du corps et celle du sang artériel. Des conditions aux limites peuvent être également fixées, par exemple température du gel ou du liquide entre la sonde et le corps du patient pour faciliter l'adaptation d'impédance acoustique.

La résolution de l'équation BHTE permet d'obtenir en chaque point de la grille l'évolution de la température au cours du temps.

On procède ensuite en 430 à l'estimation proprement dite de la dose thermique déposée en chaque point de la grille. La dose thermique peut être conventionnellement exprimée en durée équivalente d'exposition à une température de référence, soit 43°C, comme décrit dans l'article de P. Lele intitulé « Thresholds and mechanisms of ultrasonic damage to "organized" animal tissues » publié dans Symposium of Biological Effects and Characterization of Ultrasound Sources, Rockville, MD: DHEW (Pub) FDA; 78-8048, pp. 224-239, 1977.

La durée équivalente d'exposition à la température de référence au point P est donnée par :

$$d_{43}(P) = \int_{t_0}^{t_{end}} R^{(43-T_P(t))} dt$$

où $R = 0.25$ si $T_P < 43°C$ et $R = 0.5$ sinon, $t_0$ et $t_{end}$ sont respectivement l'instant de début et de fin du traitement HIFU.

Si la durée équivalente d'exposition est supérieure à une valeur de seuil prédéterminée, le tissu est considéré comme nécrosé. A défaut, il est considéré comme épargné.

D'autres critères de destruction tissulaire, par exemple de durée d'exposition au-delà d'une température de seuil pourront être envisagés par l'homme du métier sans sortir pour autant du cadre de la présente invention.

Le système de paramétrage du dispositif de traitement HIFU permet de simuler en quasi temps réel un protocole de traitement et, le cas échéant, adapter les paramètres nominaux de manière à satisfaire à un objectif défini par les contours des régions à nécroser et ceux des régions à épargner dans la zone à traiter. Le praticien peut intervenir à tout moment dans le processus itératif et ajouter des contraintes ou en relâcher d'autres. Une fois les paramètres de traitement validés, ceux-ci sont transférés dans le dispositif HIFU pour lancer le traitement.

## Revendications

1. Méthode de paramétrage d'un dispositif de traitement HIFU (150) équipé d'une sonde ultrasonore pour nécroser au moins une région tissulaire d'une zone à traiter d'un patient, ledit traitement comprenant une pluralité de tirs ultrasonores et étant **caractérisé par** des paramètres de traitement définissant notamment la position et l'orientation de la sonde à chaque tir relativement à la zone à traiter, la zone à traiter étant modélisée par différentes régions tissulaires, incluant au moins une région tissulaire à nécroser et une région tissulaire à préserver, chaque région tissulaire étant **caractérisée par** des paramètres géométriques et des paramètres physiologiques, ladite méthode de paramétrage comprenant :

   - une étape d'estimation du champ ultrasonore (220) dans la zone à traiter au moyen d'un méta-modèle utilisant une base de données dans laquelle sont stockées des cartographies précalculées de champ ultrasonore pour différentes valeurs des paramètres géométriques et physiologiques de chaque région tissulaire de ladite zone, l'estimation du champ ultrasonore étant obtenue par interpolation de cartographies précalculées ;
   - une étape d'estimation de la dose thermique (230) déposée en chaque point d'une grille au sein de la zone à traiter à partir du champ ultrasonore dans cette zone ;
   - une étape d'estimation de la réponse tissulaire (240) en chaque point de ladite grille pour déterminer si la dose thermique déposée conduit ou non à une nécrose en ce point, définissant ainsi une région nécrosée estimée et une région préservée estimée ;
   - une étape d'adaptation des paramètres de traitement (250) visant à minimiser une fonction de coût dépendant de l'écart entre la région nécrosée estimée et la région tissulaire à nécroser, sous contrainte que la région à préserver soit comprise dans la région préservée estimée.

2. Méthode de paramétrage d'un dispositif de traitement HIFU selon la revendication 1, **caractérisée en ce que** la fonction de coût inclut une somme d'une pluralité de fonctions de coût élémentaires, chacune desdites fonctions de coût élémentaire étant associée à un tir, à une région tissulaire à nécroser, ou à une sous-région d'une région tissulaire à nécroser, et/ou étant une fonction visant à minimiser ou maximiser au moins un objectif choisi parmi

   - un écart entre une région ou une sous-région de celle-ci estimée nécrosée par un ou plusieurs tirs et la région ou la sous-région de celle-ci à nécroser correspondante,
   - un volume de tissus sains nécrosés à l'issue d'un ou plusieurs tirs,
   - une durée globale d'un ou plusieurs tirs,
   - un nombre de positions de la sonde pour la réalisation d'un ensemble de tirs, notamment de la totalité des tirs,
   - une période de repos entre deux tirs consécutifs ou une somme de telles périodes de repos.

3. Méthode de paramétrage d'un dispositif de traitement HIFU selon la revendication 1 ou 2, **caractérisée en ce que** lesdits paramètres de traitement comprennent également la durée et la cadence de répétition des tirs ultrasonores.

4. Méthode de paramétrage d'un dispositif de traitement HIFU selon l'une des revendications précédentes, **caractérisée en ce que** la sonde comprend une pluralité de transducteurs et que lesdits paramètres de traitement comprennent une loi de phase et/ou de fréquence et/ou de puissance à appliquer sur l'ensemble des éléments transducteurs.

**5.** Méthode de paramétrage d'un dispositif de traitement HIFU selon l'une des revendications précédentes, **caractérisée en ce que** les paramètres physiologiques d'une région tissulaire comprennent au moins un paramètre parmi la densité, la chaleur spécifique, la conductivité thermique du tissu, la chaleur spécifique du sang et le taux de perfusion sanguine du tissu.

**6.** Méthode de paramétrage d'un dispositif de traitement HIFU selon l'une des revendications précédentes, **caractérisée en ce que** l'estimation du champ ultrasonore est obtenue dans le repère local de la sonde par interpolation desdites cartographies précalculées, la distribution du champ ultrasonore dans la zone à calculer en étant déduite par changement de repère.

**7.** Méthode de paramétrage d'un dispositif de traitement HIFU selon l'une des revendications précédentes, **caractérisée en ce que** l'estimation de la dose thermique est obtenue par une résolution d'une équation de transfert de chaleur dans les différentes régions tissulaires, prenant en compte l'énergie ultrasonore absorbée en chaque point de la grille à chaque tir ultrasonore.

**8.** Méthode de paramétrage d'un dispositif de traitement HIFU selon l'une des revendications précédentes, **caractérisée en ce que** le résultat du traitement est simulé en calculant, en chaque point de la grille, la dose thermique absorbée en ce point sous la forme d'une durée d'exposition équivalente à une température de référence, le tissu étant considéré comme nécrosé en ce point si cette durée est supérieure à une valeur de seuil prédéterminée et préservé sinon.

**9.** Méthode de paramétrage d'un dispositif de traitement HIFU selon la revendication 8, **caractérisée en ce que** la fonction de coût dépend de l'écart entre l'étendue de la région tissulaire à nécroser avec celle de la région tissulaire nécrosée estimée.

**10.** Méthode de paramétrage d'un dispositif de traitement HIFU selon l'une des revendications précédentes, **caractérisée en ce que** lors de l'étape d'adaptation des paramètres de traitement (250), les paramètres de traitement sont adaptés de manière itérative au moyen d'une descente de gradient, d'une descente de gradient stochastique ou d'un algorithme génétique.

**11.** Méthode de paramétrage d'un dispositif de traitement HIFU selon l'une des revendications précédentes, **caractérisée en ce que** pour au moins une itération d'adaptation des paramètres de traitement, la région tissulaire nécrosée estimée et/ou la région tissulaire préservée estimée sont/est visualisée(s) sur un afficheur avec la région tissulaire à nécroser et/ou la région tissulaire à préserver en superposition avec l'image de la zone à traiter.

**12.** Système de paramétrage d'un dispositif de traitement HIFU équipé d'une sonde ultrasonore pour nécroser au moins une région tissulaire d'une zone à traiter d'un patient, ledit traitement comprenant une pluralité de tirs ultrasonores et étant **caractérisé par** des paramètres de traitement définissant notamment la position et l'orientation de la sonde à chaque tir relativement à la zone à traiter, la zone à traiter étant modélisée par différentes régions tissulaires, incluant au moins une région tissulaire à nécroser et une région tissulaire à préserver, chaque région tissulaire étant **caractérisée par** des paramètres géométriques et physiologiques, ledit système de paramétrage comprenant :

- un module d'estimation du champ ultrasonore (220) dans la zone à traiter au moyen d'un méta-modèle utilisant une base de données (210) dans laquelle sont stockées des cartographies précalculées de champ ultrasonore pour différentes valeurs des paramètres géométriques et physiologiques de chaque région tissulaire de ladite zone, ledit module d'estimation estimant le champ ultrasonore par interpolation de cartographies précalculées extraites de la base de données ;
- un module d'estimation de la dose thermique (230) déposée en chaque point d'une grille au sein de la zone à traiter à partir du champ ultrasonore dans cette zone ;
- un module d'estimation de la réponse tissulaire (240) en chaque point de ladite grille pour déterminer si la dose thermique déposée conduit ou non à une nécrose en ce point, définissant ainsi une région nécrosée estimée et une région préservée estimée ;
- un module d'adaptation des paramètres de traitement (250) pour minimiser une fonction de coût dépendant de l'écart entre la région nécrosée estimée et la région tissulaire à nécroser, sous contrainte que la région à préserver soit comprise dans la région préservée estimée.

**13.** Système de paramétrage d'un dispositif de traitement HIFU selon la revendication 12, **caractérisé en ce que** lesdits paramètres de traitement comprennent également la durée et la cadence de répétition des tirs ultrasonores.

**14.** Système de paramétrage d'un dispositif de traitement HIFU selon la revendication 12 ou 13, **caractérisé en ce que** la sonde comprend une pluralité de transducteurs et que lesdits paramètres de traitement comprennent une loi de phase et/ou de fréquence et/ou de puissance à appliquer sur l'ensemble des éléments transducteurs.

## Patentansprüche

1. Parametrierungsverfahren einer HIFU-Behandlungsvorrichtung (150), die mit einer Ultraschallsonde ausgestattet ist, um mindestens einen Gewebebereich eines zu behandelnden Bereichs eines Patienten zu nekrotisieren, wobei die Behandlung eine Vielzahl von Ultraschallschüssen umfasst und durch Behandlungsparameter gekennzeichnet ist, die insbesondere die Position und die Ausrichtung der Sonde bei jedem Schuss in Bezug auf den zu behandelnden Bereich definieren, wobei der zu behandelnde Bereich durch verschiedene Gewebebereiche modelliert wird, einschließend mindestens einen zu nekrotisierenden Gewebebereich und einen zu erhaltenden Gewebebereich, wobei jeder Gewebebereich durch geometrische Parameter und physiologische Parameter gekennzeichnet ist, wobei das Parametrierungsverfahren Folgendes umfasst:

   - einen Schritt der Schätzung des Ultraschallfelds (220) in dem zu behandelnden Bereich mittels eines Metamodells unter Verwendung einer Datenbank, in der vorberechnete Ultraschallfeld-Mappings für verschiedene Werte der geometrischen und physiologischen Parameter jedes Gewebebereichs des Bereichs gespeichert sind, wobei die Schätzung des Ultraschallfelds durch Interpolation von vorberechneten Mappings erhalten wird;
   - einen Schritt der Schätzung der Wärmedosis (230), die an jedem Punkt eines Gitters innerhalb des zu behandelnden Bereichs ausgehend von dem Ultraschallfeld in diesem Bereich abgegeben wird;
   - einen Schritt der Schätzung der Gewebeantwort (240) an jedem Punkt des Gitters, um zu bestimmen, ob die abgegebene Wärmedosis an diesem Punkt zu einer Nekrose führt oder nicht, wodurch ein geschätzter nekrotischer Bereich und ein geschätzter erhaltener Bereich definiert werden;
   - einen Schritt der Anpassung der Behandlungsparameter (250), der darauf abzielt, eine Kostenfunktion zu minimieren, die von der Abweichung zwischen dem geschätzten nekrotischen Bereich und dem zu nekrotisierenden Gewebebereich abhängt, unter der Bedingung, dass der zu erhaltende Bereich in dem geschätzten erhaltenen Bereich liegt.

2. Parametrierungsverfahren einer HIFU-Behandlungsvorrichtung nach Anspruch 1, **dadurch gekennzeichnet, dass** die Kostenfunktion eine Summe einer Vielzahl von elementaren Kostenfunktionen einschließt, wobei jede dieser elementaren Kostenfunktionen mit einem Schuss, einem zu nekrotisierenden Gewebebereich oder einem Unterbereich eines zu nekrotisierenden Gewebebereichs verbunden ist, und/oder eine Funktion zur Minimierung oder Maximierung von mindestens einem Ziel ausgewählt aus

   - einer Abweichung zwischen einem Bereich oder einem Teilbereich davon, der durch einen oder mehrere Schüsse als nekrotisch eingeschätzt wird, und dem entsprechenden nekrotischen Bereich oder Teilbereich davon,
   - einem Volumen an gesundem Gewebe, das nach einem oder mehreren Schüssen nekrotisch ist,
   - einer Gesamtdauer von einem oder mehreren Schüssen,
   - einer Anzahl von Sondenpositionen für die Durchführung einer Reihe von Schüssen, insbesondere aller Schüsse,
   - einer Ruhezeit zwischen zwei aufeinanderfolgenden Schüssen oder eine Summe solcher Ruhezeiten, ist.

3. Parametrierungsverfahren einer HIFU-Behandlungsvorrichtung nach Anspruch 1 oder 2, **dadurch gekennzeichnet, dass** die Behandlungsparameter auch die Dauer und die Wiederholrate der Ultraschallschüsse umfassen.

4. Parametrierungsverfahren einer HIFU-Behandlungsvorrichtung nach einem der vorhergehenden Ansprüche, **dadurch gekennzeichnet, dass** die Sonde eine Vielzahl von Wandlern umfasst und dass die Behandlungsparameter ein auf alle Wandlerelemente anzuwendendes Phasen- und/oder Frequenz- und/oder Leistungsgesetz umfassen.

5. Parametrierungsverfahren einer HIFU-Behandlungsvorrichtung nach einem der vorhergehenden Ansprüche, **dadurch gekennzeichnet, dass** die physiologischen Parameter eines Gewebebereichs mindestens einen Parameter aus der Dichte, der spezifischen Wärme, der Wärmeleitfähigkeit des Gewebes, der spezifischen Wärme des Blutes und der Blutperfusionsrate des Gewebes umfassen.

6. Parametrierungsverfahren einer HIFU-Behandlungsvorrichtung nach einem der vorhergehenden Ansprüche, **dadurch gekennzeichnet, dass** die Schätzung des Ultraschallfelds in der lokalen Referenzlinie der Sonde durch Interpolation der vorberechneten Mappings erhalten wird, wobei die Verteilung des Ultraschallfelds in dem zu berechnenden Bereich durch Änderung der Referenzlinie abgeleitet wird.

7. Parametrierungsverfahren einer HIFU-Behandlungsvorrichtung nach einem der vorhergehenden Ansprüche, **dadurch gekennzeichnet, dass** die Schätzung der Wärmedosis durch eine Auflösung einer Wärmeübertragungsgleichung in den verschiedenen Gewebebereichen erreicht wird, wobei die an jedem Punkt des Gitters bei jedem Ultraschallschuss absorbierte Ultraschallenergie berücksichtigt wird.

8. Parametrierungsverfahren einer HIFU-Behandlungsvorrichtung nach einem der vorhergehenden Ansprüche, **dadurch gekennzeichnet, dass** das Behandlungsergebnis simuliert wird, indem an jedem Punkt des Gitters die an diesem Punkt absorbierte Wärmedosis in Form einer Expositionsdauer berechnet wird, die einer Referenztemperatur entspricht, wobei das Gewebe an diesem Punkt als nekrotisch angesehen wird, wenn diese Dauer über einem vorbestimmten Schwellenwert liegt und andernfalls erhalten bleibt.

9. Parametrierungsverfahren einer HIFU-Behandlungsvorrichtung nach Anspruch 8, **dadurch gekennzeichnet, dass** die Kostenfunktion von der Abweichung zwischen dem Umfang des zu nekrotisierenden Gewebebereichs und dem des geschätzten nekrotischen Gewebebereichs abhängt.

10. Parametrierungsverfahren einer HIFU-Behandlungsvorrichtung nach einem der vorhergehenden Ansprüche, **dadurch gekennzeichnet, dass** bei dem Schritt der Anpassung der Behandlungsparameter (250) die Behandlungsparameter mittels eines Gradientenabfalls, eines stochastischen Gradientenabfalls oder eines genetischen Algorithmus iterativ angepasst werden.

11. Parametrierungsverfahren einer HIFU-Behandlungsvorrichtung nach einem der vorhergehenden Ansprüche, **dadurch gekennzeichnet, dass** für mindestens eine Iteration der Anpassung der Behandlungsparameter der geschätzte nekrotische Gewebebereich und/oder der geschätzte erhaltene Gewebebereich auf einer Anzeige mit dem zu nekrotisierenden Gewebebereich und/oder dem zu erhaltenden Gewebebereich in Überlagerung mit dem Bild des zu behandelnden Bereichs visualisiert wird/werden.

12. Parametrierungssystem einer HIFU-Behandlungsvorrichtung, die mit einer Ultraschallsonde ausgestattet ist, um mindestens einen Gewebebereich eines zu behandelnden Bereichs eines Patienten zu nekrotisieren, wobei die Behandlung eine Vielzahl von Ultraschallschüssen umfasst und durch Behandlungsparameter gekennzeichnet ist, die insbesondere die Position und die Ausrichtung der Sonde bei jedem Schuss in Bezug auf den zu behandelnden Bereich definieren, wobei der zu behandelnde Bereich durch verschiedene Gewebebereiche modelliert wird, einschließend mindestens einen zu nekrotisierenden Gewebebereich und einen zu erhaltenden Gewebebereich, wobei jeder Gewebebereich durch geometrische und physiologische Parameter gekennzeichnet ist, wobei das Parametrierungssystem Folgendes umfasst:

- ein Modul zur Schätzung des Ultraschallfelds (220) in dem zu behandelnden Bereich mittels eines Metamodells unter Verwendung einer Datenbank (210), in der vorberechnete Ultraschallfeld-Mappings für verschiedene Werte der geometrischen und physiologischen Parameter jedes Gewebebereichs des Bereichs gespeichert sind, wobei das Modul zur Schätzung das Ultraschallfeld durch Interpolation von vorberechneten Mappings, die aus der Datenbank extrahiert wurden, schätzt;
- ein Modul zur Schätzung der Wärmedosis (230), die an jedem Punkt eines Gitters innerhalb des zu behandelnden Bereichs ausgehend von dem Ultraschallfeld in diesem Bereich abgegeben wird;
- ein Modul zur Schätzung der Gewebeantwort (240) an jedem Punkt des Gitters, um zu bestimmen, ob die abgegebene Wärmedosis an diesem Punkt zu einer Nekrose führt oder nicht, wodurch ein geschätzter nekrotisierter Bereich und ein geschätzter erhaltener Bereich definiert werden;
- ein Modul zur Anpassung der Behandlungsparameter (250), um eine Kostenfunktion zu minimieren, die von der Abweichung zwischen dem geschätzten nekrotischen Bereich und dem zu nekrotisierenden Gewebebereich abhängt, unter der Bedingung, dass der zu erhaltende Bereich in dem geschätzten erhaltenen Bereich liegt.

13. Parametrierungssystem einer HIFU-Behandlungsvorrichtung nach Anspruch 12, **dadurch gekennzeichnet, dass** die Behandlungsparameter auch die Dauer und die Wiederholrate der Ultraschallschüsse umfassen.

14. Parametrierungssystem einer HIFU-Behandlungsvorrichtung nach Anspruch 12 oder 13, **dadurch gekennzeichnet, dass** die Sonde eine Vielzahl von Wandlern umfasst und dass die Behandlungsparameter ein Phasen- und/oder Frequenz- und/oder Leistungsgesetz umfassen, das auf alle Wandlerelemente anzuwenden ist.

**Claims**

1.  Method for parameterising a HIFU treatment device (150) equipped with an ultrasound probe for necrotising at least one tissue region of an area to be treated of a patient, said treatment comprising a plurality of ultrasound shots and being **characterised by** treatment parameters defining in particular the position and orientation of the probe at each shot relative to the area to be treated, the area to be treated being modelled by different tissue regions, including at least one tissue region to be necrotised and one tissue region to be preserved, each tissue region being **characterised by** geometric parameters and physiological parameters, said parameterisation method comprising:

    - a step (220) of estimating the ultrasonic field in the area to be treated by means of a metamodel using a database in which precomputed ultrasonic field maps are stored for different values of the geometric and physiological parameters of each tissue region of said area, the ultrasonic field estimate being obtained by interpolation of precomputed maps;
    - a step (230) of estimating the thermal dose applied at each point on a grid within the area to be treated from the ultrasonic field in this area;
    - a step (240) of estimating the tissue response at each point on said grid to determine whether or not the thermal dose applied leads to necrosis at this point, thus defining an estimated necrotised region and an estimated preserved region;
    - a step (250) of adjusting the treatment parameters in order to minimise a cost function that is dependent on the difference between the estimated necrotised region and the tissue region to be necrotised, with the condition that the region to be preserved lies within the estimated preserved region.

2.  Method for parameterising a HIFU treatment device according to claim 1, **characterised in that** the cost function includes a sum of a plurality of unit cost functions, each of said unit cost functions being associated with a shot, with a tissue region to be necrotised, or with a sub-region of a tissue region to be necrotised, and/or being a function intended to minimise or maximise at least one objective chosen from among

    - a difference between a region or sub-region thereof estimated to have been necrotised by one or more shots and the corresponding region or sub-region thereof to be necrotised,
    - a volume of healthy tissue necrotised after one or more shots,
    - an overall duration of one or more shots,

    - a number of positions of the probe for carrying out a set of shots, in particular all of the shots,
    - a rest period between two consecutive shots or a sum of such rest periods.

3.  Method for parameterising a HIFU treatment device according to claim 1 or 2, **characterised in that** said treatment parameters further comprise the duration and repetition rate of the ultrasound shots.

4.  Method for parameterising a HIFU treatment device according to one of the preceding claims, **characterised in that** the probe comprises a plurality of transducers, and **in that** said treatment parameters comprise a phase and/or frequency and/or power law to be applied to all of the transducer elements.

5.  Method for parameterising a HIFU treatment device according to one of the preceding claims, **characterised in that** the physiological parameters of a tissue region include at least one parameter of density, specific heat, tissue thermal conductivity, specific heat of blood or tissue blood perfusion rate.

6.  Method for parameterising a HIFU treatment device according to one of the preceding claims, **characterised in that** the ultrasonic field is estimated in the local reference frame of the probe by interpolating said pre-computed maps, the distribution of the ultrasonic field in the area to be computed being deduced by changing the reference frame.

7.  Method for parameterising a HIFU treatment device according to one of the preceding claims, **characterised in that** the thermal dose is estimated by solving a heat transfer equation in the different tissue regions, taking into account the ultrasound energy absorbed at each point on the grid during each ultrasound shot.

8.  Method for parameterising a HIFU treatment device according to one of the preceding claims, **characterised in that** the result of the treatment is simulated by computing, at each point on the grid, the thermal dose absorbed at that point in the form of an exposure time equivalent to a reference temperature, the tissue being considered necrotised at that point if that time exceeds a predetermined threshold value, and preserved otherwise.

9.  Method for parameterising a HIFU treatment device according to claim 8, **characterised in that** the cost function depends on the difference between the extent of the tissue region to be necrotised and the extent of the estimated necrotised tissue region.

10. Method for parameterising a HIFU treatment device according to one of the preceding claims, **charac-**

**terised in that** during the step (250) of adjusting the treatment parameters, the treatment parameters are adjusted iteratively using gradient descent, stochastic gradient descent or a genetic algorithm.

11. Method for parameterising a HIFU treatment device according to one of the preceding claims, **characterised in that** for at least one treatment parameter adjustment iteration, the estimated necrotised tissue region and/or the estimated preserved tissue region is/are displayed on a display with the tissue region to be necrotised and/or the tissue region to be preserved overlaying the image of the area to be treated.

12. System for parameterising a HIFU treatment device equipped with an ultrasound probe for necrotising at least one tissue region of an area to be treated of a patient, said treatment comprising a plurality of ultrasound shots and being **characterised by** treatment parameters defining in particular the position and orientation of the probe at each shot relative to the area to be treated, the area to be treated being modelled by different tissue regions, including at least one tissue region to be necrotised and one tissue region to be preserved, each tissue region being **characterised by** geometric and physiological parameters, said parameterisation system comprising:

   - a module (220) for estimating the ultrasonic field in the area to be treated by means of a metamodel using a database (210) in which precomputed ultrasonic field maps are stored for different values of the geometric and physiological parameters of each tissue region of said area, said estimation module estimating the ultrasonic field by interpolation of precomputed maps extracted from the database;
   - a module (230) for estimating the thermal dose applied at each point on a grid within the area to be treated from the ultrasonic field in this area;
   - a module (240) for estimating the tissue response at each point on said grid to determine whether or not the thermal dose applied leads to necrosis at this point, thus defining an estimated necrotised region and an estimated preserved region;
   - a module (250) for adjusting the treatment parameters in order to minimise a cost function that is dependent on the difference between the estimated necrotised region and the tissue region to be necrotised, with the condition that the region to be preserved lies within the estimated preserved region.

13. System for parameterising a HIFU treatment device according to claim 12, **characterised in that** said treatment parameters further comprise the duration and repetition rate of the ultrasound shots.

14. System for parameterising a HIFU treatment device according to claim 12 or 13, **characterised in that** the probe comprises a plurality of transducers, and **in that** said treatment parameters comprise a phase and/or frequency and/or power law to be applied to all of the transducer elements.

100

120

110

130

GUI

dispositif d'imagerie

HMI

140

module de simulation

150

dispositif
HIFU

160

# Fig. 1

210

module de prédiction de la
du champ ultrasonore

220

module d'estimation de la
distribution de la dose
thermique déposée

230

module d'estimation de la
réponse tissulaire

240

module d'adaptation des
paramètres du traitement
HIFU

250

# Fig. 2

modèle de la zone à traiter
paramètres physiologiques et
géométriques des différentes régions
tissulaires

310

position, orientation, configuration et
paramétrage de la sonde HIFU

320

pour chaque tir obtention de cartographies
de champ ultrasonore précalculées stockées
dans la base de données

330

estimation du champ ultrasonore dans le
repère local de la sonde par interpolation des
cartographies obtenues

340

transformation par changement de repère

350

# Fig. 3

obtention de l'énergie acoustique
apportée à chaque tir en chaque point
de la zone à traiter — 410

résolution de l'équation de la chaleur
par différences finies dans la zone à traiter
Obtention de la cartographie
de température au cours du temps — 420

calcul de la dose thermique déposée à
partir de l'évolution de la cartographie
de température au cours du temps — 430

# Fig. 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20210000541 A

- US 2014296842 A1

**Littérature non-brevet citée dans la description**

- **S. CHATILLON et al.** Applications of intensive HIFU simulation based on surrogate models using the CIVA HealthCare platform. *J. of Phys.*, 2021, 1761
- **J. CHATO**. Fundamentals of Bioheat Transfer. Springer, 1990

- **P. LELE**. Thresholds and mechanisms of ultrasonic damage to "organized" animal tissues. *Symposium of Biological Effects and Characterization of Ultrasound Sources*, 1977, vol. 78-8048, 224-239